# EUROPÄISCHE PATENTSCHRIFT

(11) **EP 1 192 212 B1**
(45) Veröffentlichungstag und Bekanntmachung des Hinweises auf die Patenterteilung: **04.01.2006**
(21) Anmeldenummer: 00945771.4
(22) Anmeldetag: 16.06.2000
(51) Int. Cl.: C08K 5/12, C08K 5/092

(54) **VERWENDUNG VON CYCLOHEXANPOLYCARBONSÄUREN ALS WEICHMACHER ZUR HERSTELLUNG TOXIKOLOGISCH GÜNSTIG ZU BEWERTENDER KUNSTSTOFFE**
USE OF CYCLOHEXANE POLYCARBOXYLIC ACIDS AS PLASTICIZERS IN THE PRODUCTION OF PLASTICS WHICH ARE RATED TOXICOLOGICALLY FAVOURABLE
UTILISATION D'ACIDES CYCLOHEXANE POLYCARBOXYLIQUES COMME PLASTIFIANTS POUR LA PRODUCTION DE MATIERES PLASTIQUES DONT LA VALORISATION EST FAVORABLE SUR LE PLAN TOXICOLOGIQUE

(30) Priorität: 18.06.1999 DE 19927977
(43) Veröffentlichungstag der Anmeldung: 03.04.2002
(73) Patentinhaber: BASF Aktiengesellschaft, 67056 Ludwigshafen (DE)
(72) Erfinder: BRUNNER, Melanie, D-68165 Mannheim (DE); THIL, Lucien, D-67117 Limburgerhof (DE); BREITSCHEIDEL, Boris, D-67117 Limburgerhof (DE); OTTER, Rainer, D-69207 Sandhausen (DE)
(74) Vertreter: Isenbruck, Günter
(86) Internationale Anmeldenummer: PCT/EP2000/005609
(87) Internationale Veröffentlichungsnummer: WO 2000/078853

(56) Entgegenhaltungen:
- EP-A- 0 922 553
- WO-A-99/32427
- DE-B- 1 263 296
- PATENT ABSTRACTS OF JAPAN vol. 1995, no. 04, 31. Mai 1995 (1995-05-31) & JP 07 011074 A (NEW JAPAN CHEM CO LTD), 13. Januar 1995 (1995-01-13)
- PATENT ABSTRACTS OF JAPAN vol. 1995, no. 10, 30. November 1995 (1995-11-30) & JP 07 173342 A (NEW JAPAN CHEM CO LTD), 11. Juli 1995 (1995-07-11)

## Beschreibung

Die vorliegende Erfindung betrifft die Verwendung von toxikologisch günstig zu bewertenden Cyclohexanpolycarbonsäuren und Derivaten davon, wie z.B. Estern und/oder Anhydriden, als Weichmacher zur Herstellung toxikologisch günstig zu bewertender Kunststoffe.

Bislang wurden als Weichmacher in Kunststoffen, wie z.B. PVC, sehr häufig Phthalsäureester, wie z.B. Dibutyl-, Dioctyl- oder Diisononylester der Phthalsäure, verwendet, wie dies z.B. aus der FR-A 23 97 131 hervorgeht. Diesen wird jedoch seit kurzer Zeit nachgesagt, daß sie gesundheitlich nicht unbedenklich sind, so daß ihre Verwendung in Kunststoffen zur Herstellung von z.B. Kinderspielzeug immer stärker in der Kritik steht und in einigen Ländern bereits verboten ist. Im Tierversuch wurde mittlerweise gezeigt, daß Phthalate zu einer Peroxisomenproliferation führen können, welche in ursächlichem Zusammenhang mit den bei Maus und Ratte in Langzeitstudien aufgetretenen Lebertumoren steht.

Die Verwendung von einigen Cyclohexan-1,2-dicarbonsäureestern als Weichmacher ist ebenfalls aus dem Stand der Technik bekannt. So ist die Verwendung von Cyclohexandicarbonsäuredimethyl oder -diethylestem (DE-A 28 23 165) und Cyclohexan-1,2-dicarbonsäuredi(2-ethylhexyl)ester (DE-A 12 63 296), als Weichmacher in Kunststoffen beschrieben.

In PCT/EP 98/08346 wird offenbart, daß Cyclohexanpolycarbonsäuren und Derivate davon als Weichmacher verwendet können. In diesem Zusammenhang wird offenbart, daß Cyclohexanpolycarbonsäuren und Derivate davon im Vergleich mit den bislang hauptsächlich als Weichmacher verwendeten Phthalaten eine niedrigere Dichte und Viskosität aufweisen und darüber hinaus u.a. auch zu einer Verbesserung der Kälteflexibilität des Kunststoffs gegenüber der Verwendung der entsprechenden Phthalate als Weichmacher führen. Des weiteren wird in PCT/EP 98/08346 offenbart, daß Cyclohexanpolycarbonsäuren und Derivate davon ein besseres Verarbeitungsverhalten im Dry-Blend und als Folge eine erhöhte Produktionsgeschwindigkeit sowie in Plastisol-Verarbeitungen Vorteile durch eine deutlich niedrigere Viskosität gegenüber den entsprechenden Phthalaten aufweisen. Toxikologische Eigenschaften von Cyclohexanpolycarbonsäuren und Derivaten davon werden in PCT/EP 98/08346 mit keinem Wort erwähnt.

Der vorliegenden Erfindung lag daher die primäre Aufgabe zugrunde, Substanzen für die Verwendung als Weichmacher in Kunststoffen bereitzustellen, welche nicht nur aufgrund ihrer physikalischen und stofflichen Eigenschaften für eine Verwendung als Weichmacher in Kunststoffen geeignet sind, sondern darüber hinaus auch aufgrund ihrer toxikologischer Eigenschaften für eine derartige Verwendung als geeignet einzustufen sind.

Die im Zusammenhang mit Phthalaten beobachtete Entstehung von Lebertumoren in Nagern scheint über den Peroxisomenproliferator-aktivierten Rezeptor-α (PPARα; *peroxisome proliferator-activated receptor-α)* vermittelt zu werden. Die diesem Mechanismus zugrunde liegende Peroxisomenproliferation läßt sich mittels verschiedener Indikatoren, unter anderem anhand eines deutlichen Anstiegs des absoluten bzw. relativen Lebergewichtes sowie anhand eines Anstiegs bestimmter Enzymaktivitäten, etwa der spezifischen Aktivität der cyanid-insensitiven Palmitoyl-CoA Oxidase (Pal-CoA Oxidase), nachweisen.

Der vorliegenden Erfindung liegt der überraschende Befund zugrunde, daß Cyclohexanpolycarbonsäuren und Derivate davon, d.h. kernhydrierte Benzolpolycarbonsäuren und Derivate davon, im Gegensatz zu verschiedenen herkömmlichen Weichmachern, insbesondere Phthalaten oder Phthalsäurederivaten, keine biologisch signifikante Peroxisomenproliferation bedingen und somit nicht nur im Hinblick auf ihre physikalischen und stofflichen Eigenschaften, sondern auch in toxikologischer Hinsicht im Vergleich mit diesen herkömmlichen Weichmachern als günstig zu bewerten sind.

Im Sinne der vorliegenden Erfindung gilt eine Cyclohexanpolycarbonsäure oder ein Derivat davon oder ein Gemisch aus zwei oder mehr davon insbesondere dann als toxikologisch günstig, wenn im Tierversuch mit Nagern, die über eine Dauer von mindestens 14 Tagen täglich eine orale Dosis von 1000 mg / kg Körpergewicht dieser Cyclohexanpolycarbonsäure oder des Derivats davon oder des Gemischs aus zwei oder mehr davon per Schlundsonde erhalten haben, im Vergleich mit den entsprechenden unbehandelten Kontrolltieren weder ein statistisch signifikanter Anstieg des absoluten Lebergewichts oder des relativen Lebergewichts, d.h. des auf das Gesamtkörpergewicht bezogenen Lebergewichts, noch ein toxikologisch relevanter Anstieg der spezifischen Enzymaktivität der cyanid-insensitiven Palmitoyl-CoA Oxidase festgestellt wird.

Ein statistisch signifikanter Anstieg des absoluten oder relativen Lebergewichts liegt z.B. bei Ratten üblicherweise dann vor, wenn ein Anstieg des absoluten oder relativen Lebergewichts eines Versuchstieres, das über die Dauer von mindestens 14 Tagen täglich eine orale Dosis von 1000 mg Prüfsubstanz / kg Körpergewicht per Schlundsonde erhalten hat, festgestellt wird, der mehr als 10% über dem Anstieg des absoluten oder relativen Lebergewichts eines entsprechenden unbehandelten Kontrolltieres liegt.

Ein statistisch signifikanter Anstieg des absoluten oder relativen Lebergewichts im Sinne der vorliegenden Erfindung liegt insbesondere dann vor, wenn der statistische Anstieg des absoluten oder relativen Lebergewichts des Versuchstieres gegenüber dem des unbehandelten Kontrolltieres von mehr als 10% mittels des Dunnett-Tests bestimmt wurde (Dunnett, C.W. (1955), A multiple comparison procedure for comparing several treatments with a control, J. Am. Stat. Assoc. 50, 1096-1121; Dunnett, C.W. (1964), New tables for multiple comparisons with a control, Biometrics, 20, 482-491).

Ein toxikologisch relevanter Anstieg der spezifischen Aktivität der cyanid-insensitiven Palmitoyl-CoA Oxidase liegt dann vor, wenn die spezifische Aktivität der cyanid-insensitiven Palmitoyl-CoA Oxidase, die im Leberhomogenat eines Versuchstieres gemessen wurde, welches über die Dauer von mindestens 14 Tagen täglich eine orale Dosis von 1000 mg Prüfsubstanz / kg Körpergewicht per Schlundsonde erhalten hat, mehr als doppelt so hoch ist wie die im Leberhomogenat eines unbehandelten Kontrolltieres bestimmte spezifische Aktivität der cyanid-insensitiven Palmitoyl-CoA Oxidase.

Üblicherweise wird die spezifische Aktivität [mU/mg Protein] der cyanid-insensitiven Palmitoyl-CoA Oxidase nach Lazarow (1981), Enzymology 72, 315-319, bestimmt, wobei die Bestimmung der Proteinmenge im Leberhomogenat routinemäßig nach dem Fachmann wohlbekannten Proteinbestimmungsmethoden, etwa der Lowry-Methode, durchgeführt wird.

Darüber hinaus ist bei den im Sinne der vorliegenden Erfindung toxikologisch als günstig zu bewertenden Cyclohexanpolycarbonsäuren und Derivaten davon zu erwarten, daß auch in bezug auf reproduktionstoxikologische Parameter relevante Verbesserungen gegenüber herkömmlichen Weichmachern, insbesondere den sehr häufig zu diesem Zweck verwendeten Phthalaten und Phthalsäurederivaten, erreicht werden.

Demzufolge betrifft die vorliegende Erfindung die Verwendung einer Cyclohexanpolycarbonsäure oder eines Derivats davon oder eines Gemischs aus zwei oder mehr davon, welche(s) im Tierversuch mit Nagern bei einer täglichen oralen Dosierung von 1000 mg / kg Körpergewicht der entsprechenden Cyclohexanpolycarbonsäure oder des Derivats davon oder des Gemischs aus zwei oder mehr davon per Schlundsonde über die Dauer von mindestens 14 Tagen im Vergleich zu unbehandelten Kontrolltieren weder zu einem signifikanten Anstieg des Lebergewichtes nach der Behandlung noch zu einer Verdoppelung der im Leberhomogenat gemessenen spezifischen Aktivität der cyanid-insensitiven Palmitoyl-CoA Oxidase führt, als Weichmacher zur Herstellung toxikologisch günstig zu bewertender Kunststoffe.

Der erfindungsgemäß verwendete Begriff *"Cyclohexanpolycarbonsäuren und Derivate davon"* umfaßt sowohl die jeweiligen Cyclohexanpolycarbonsäuren an sich sowie Derivate davon, wobei insbesondere Mono-, Di- oder ggf. Tri- oder Tetraester sowie Anhydride der Cyclohexanpolycarbonsäuren zu nennen sind. Die eingesetzten Ester sind Alkyl-, Cykloalkyl- sowie Alkoxyalkylester, wobei die Alkyl-, Cycloalkyl- sowie Alkoxyalkylgruppen in der Regel 1 bis 30, vorzugsweise 2 bis 20 und besonders bevorzugt 3 bis 18 Kohlenstoffatome umfassen und verzweigt oder linear sein können.

### Im einzelnen sind zu nennen:

Cyclohexan-1,4-dicarbonsäurealkylester, wie z.B. Cyclohexan-1,4-dicarbonsäuremonomethylester, Cyclohexan-1,4-dicarbonsäuredimethylester, Cyclohexan-1,4-dicarbonsäurediethylester, Cyclohexan-1,4-dicarbonsäuredi-n-propylester, Cyclohexan-1,4-dicarbonsäuredi-n-butylester, Cyclohexan-1,4-dicarbonsäuredi-tert-butylester, Cyclohexan-1,4-dicarbonsäurediisobutylester, Cyclohexan-1,4-dicarbonsäuremonoglykolester, Cyclohexan-1,4-dicarbonsäurediglykolester, Cyclohexan-1,4-dicarbonsäuredi-n-octylester, Cyclohexan-1,4-dicarbonsäurediisooctylester, Cyclohexan-1,4-dicarbonsäuremono-2-ethylhexylester, Cyclohexan-1,4-dicarbonsäuredi-2-ethylhexylester, Cyclohexan-1,4-dicarbonsäuredi-n-nonylester, Cyclohexan-1,4-dicarbonsäurediisononylester, Cyclohexan-1,4-dicarbonsäuredi-n-decylester, Cyclohexan-1,4-dicarbonsäuredi-n-undecylester, Cyclohexan-1,4-dicarbonsäurediisodecylester, Cyclohexan-1,4-dicarbonsäurediisododecylester, Cyclohexan-1,4-dicarbonsäuredi-n-octadecylester, Cyclohexan-1,4-dicarbonsäurediisooctadecylester, Cyclohexan-1,4-dicarbonsäuredi-n-eicosylester, Cyclohexan-1,4-dicarbonsäuremonocyclohexylester, Cyclohexan-1,4-dicarbonsäuredicyclohexylester; Cyclohexan-1,2-dicarbonsäurealkylester, wie z.B. Cyclohexan-1,2-dicarbonsäuremonomethylester, Cyclohexan-1,2-dicarbonsäuredimethylester, Cyclohexan-1,2-dicarbonsäurediethylester, Cyclohexan-1,2-dicarbonsäuredi-n-propylester, Cyclohexan-1,2-dicarbonsäuredi-n-butylester, Cyclohexan-1,2-dicarbonsäuredi-tert.-butylester, Cyclohexan-1,2-dicarbonsäurediisobutylester, Cyclohexan-1,2-dicarbonsäuremonoglykolester, Cyclohexan-1,2-dicarbonsäurediglykolester, Cyclohexan-1,2-dicarbonsäuredi-n-octylester, Cyclohexan-1,2-dicarbonsäurediisooctylester, Cyclohexan-1,2-dicarbonsäuredi-2-ethylhexylester, Cyclohexan-1,2-dicarbonsäuredi-n-nonylester, Cyclohexan-1,2-dicarbonsäurediisononylester, Cyclohexan-1,2-dicarbonsäuredi-n-decylester, Cyclohexan-1,2-dicarbonsäurediisodecylester, Cyclohexan-1,2-dicarbonsäuredi-n-undecylester, Cyclohexan-1,2-dicarbonsäurediisododecylester, Cyclohexan-1,2-dicarbonsäuredi-n-octadecylester, Cyclohexan-1,2-dicarbonsäurediisooctadecylester, Cyclohexan-1,2-dicarbonsäuredi-n-eicosylester, Cyclohexan-1,2-dicarbonsäuremonocyclohexylester, Cyclohexan-1,2-dicarbonsäuredicyclohexylester;

Cyclohexan-1,3-dicarbonsäurealkylester, wie z.B. Cyclohexan-1,3-dicarbonsäuremonomethylester, Cyclohexan-1,3-dicarbonsäuredimethylester, Cyclohexan-1,3-dicarbonsäurediethylester, Cyclohexan-1,3-dicarbonsäuredi-n-propylester, Cyclohexan-1,3-dicarbonsäuredi-n-butylester, Cyclohexan-1,3-dicarbonsäuredi-tert.-butylester, Cyclohexan-1,3-dicarbonsäurediisobutylester, Cyclohexan-1,3-dicarbonsäuremonoglykolester, Cyclohexan-1,3-dicarbonsäurediglykolester, Cyclohexan-1,3-dicarbonsäuredi-n-octylester, Cyclohexan-1,3-dicarbonsäurediisooctylester, Cyclohexan-1,3-dicarbonsäuredi-2-ethylhexylester, Cyclohexan-1,3-dicarbonsäuredi-n-nonylester, Cyclohexan-1,3 -dicarbonsäurediisononylester, Cyclohexan-1,3-dicarbonsäuredi-n-decylester, Cyclohexan-1,3-dicarbonsäurediisodecylester, Cyclohexan-1,3-dicarbonsäuredi-n-undecylester, Cyclohexan-1,3-dicarbonsäurediisododecylester, Cyclohexan-1,3-dicarbonsäuredi-n-octadecylester, Cyclohexan-1,3-dicarbonsäurediisooctadecylester, Cyclohexan-1,3-dicarbonsäuredi-n-eicosylester, Cyclohexan-1,3-dicarbonsäuremonocyclohexylester, Cyclohexan-1,3-dicarbonsäuredicyclohexylester.

Cyclohexan-1,2,4-tricarbonsäurealkylester, wie z.B. Cyclohexan-1,2,4-tricarbonsäuremonomethylester, Cyclohexan-1,2,4-tricarbonsäuredimethylester, Cyclohexan-1,2,4-tricarbonsäurediethylester, Cyclohexan-1,2,4-tricarbonsäuredi-n-propylester, Cyclohexan-1,2,4-tricarbonsäuredi-n-butylester, Cyclohexan-1,2,4-tricarbonsäureditert-butylester, Cyclohexan-1,2,4-tricarbonsäurediisobutylester, Cyclohexan-1,2,4-tricarbonsäuremonoglykolester, Cyclohexan-1,2,4-tricarbonsäurediglykolester, Cyclohexan-1,2,4-tricarbonsäuredi-n-octylester, Cyclohexan-1,2,4-tricarbonsäurediisooctylester, Cyclohexan-1,2,4-tricarbonsäuredi-2-ethylhexylester, Cyclohexan-1,2,4-tricarbonsäuredi-n-nonylester, Cyclohexan-1,2,4-tricarbonsäurediisononylester, Cyclohexan-1,2,4-tricarbonsäuredi-n-decylester, Cyclohexan-1,2,4-tricarbonsäurediisodecylester, Cyclohexan-1,2,4-tricarbonsäuredi-n-undecylester, Cyclohexan-1,2,4-tricarbonsäurediisododecylester, Cyclohexan-1,2,4-tricarbonsäuredi-n-octadecyl-ester, Cyclohexan-1,2,4-tricarbonsäurediisooctadecylester, Cyclohexan-1,2,4-tricarbonsäuredi-n-eicosylester, Cyclohexan-1,2,4-tricarbonsäuremonocyclohexylester, Cyclohexan-1,2,4-tricarbonsäuredicyclohexylester sowie Cyclohexan-1,2,4-tricarbonsäuretrimethylester, Cyclohexan-1,2,4-tricarbonsäuretriethylester, Cyclohexan-1,2,4-tricarbonsäuretri-n-propylester, Cyclohexan-1,2,4-tricarbonsäuretri-n-butylester, Cyclohexan-1,2,4-tricarbonsäuretri-tert-butylester, Cyclohexan-1,2,4-tricarbonsäuretriisobutylester, Cyclohexan-1,2,4-tricarbonsäuretriglykolester, Cyclohexan-1,2,4-tricarbonsäuretri-n-octylester, Cyclohexan-1,2,4-tricarbonsäuretriisooctylester, Cyclohexan-1,2,4-tricarbonsäuretri-2-ethylhexylester, Cyclohexan-1,2,4-tricarbonsäuretri-n-nonylester, Cyclohexan-1,2,4-tricarbonsäuretriisododecylester, Cyclohexan-1,2,4-tricarbonsäuretri-n-undecylester, Cyclohexan-1,2,4-tricarbonsäuretriisododecylester, Cyclohexan-1,2,4-tricarbonsäuretri-n-octadecylester, Cyclohexan-1,2,4-tricarbonsäuretriisooctadecylester, Cyclohexan-1,2,4-tricarbonsäuretrin-eicosylester, Cyclohexan-1,2,4-tricarbonsäuretricyclohexylester.

Cyclohexan-1,3,5-tricarbonsäurealkylester, wie z.B. Cyclohexan-1,3,5-tricarbonsäuremonomethylester, Cyclohexan-1,3,5-tricarbonsäuredimethylester, Cyclohexan-1,3,5-tricarbonsäurediethylester, Cyclohexan-1,3,5-tricarbonsäuredi-n-propylester, Cyclohexan-1,3,5-tricarbonsäuredi-n-butylester, Cyclohexan-1,3,5-tricarbonsäureditert-butylester, Cyclohexan-1,3,5-tricarbonsäurediisobutylester, Cyclohexan-1,3,5-tricarbonsäuremonoglykolester, Cyclohexan-1,3,5-tricarbonsäurediglykolester, Cyclohexan-1,3,5-tricarbonsäuredi-n-octylester, Cyclohexan-1,3,5-tricarbonsäurediisooctylester, Cyclohexan-1,3,5-tricarbonsäuredi-2-ethylhexylester, Cyclohexan-1,3,5-tricarbonsäuredi-n-nonylester, Cyclohexan-1,3,5-tricarbonsäurediisononylester, Cyclohexan-1,3,5-tricarbonsäuredi-n-decylester, Cyclohexan-1,3,5-tricarbonsäurediisodecylester, Cyclohexan-1,3,5-tricarbonsäuredi-n-undecylester, Cyclohexan-1 ,3,5-tricarbonsäurediisododecylester, Cyclohexan-1,3,5-tricarbonsäuredi-n-octadecylester, Cyclohexan-1,3,5-tricarbonsäurediisooctadecylester, Cyclohexan-1,3,5-tricarbonsäuredi-n-eicosylester, Cyclohexan-1,3,5-tricarbonsäuremonocyclohexylester, Cyclohexan-1,3,5-tricarbonsäuredicyclohexylester, sowie Cyclohexan-1,3,5-tricarbonsäuretrimethylester, Cyclohexan-I,3,5-tricarbonsäuretriethylester, Cyclohexan-1,3,5-tricarbonsäuretri-n-propylester, Cyclohexan-1,3,5-tricarbonsäuretri-n-butylester, Cyclohexan-1,3,5-tricarbonsäuretri-tert-butylester, Cyclohexan-1,3,5-tricarbonsäuretrüsobutylester, Cyclohexan-1,3,5-tricarbonsäuretriglykolester, Cyclohexan-1,3,5-tricarbonsäuretri-n-octylester, Cyclohexan-1,3,5-tricarbonsäuretriisooctylester, Cyclohexan-1,3,5-tricarbonsäuretri-2-ethylhexylester, Cyclohexan-1,3,5-tricarbonsäuretri-n-nonylester, Cyclohexan-1,3,5-tricarbonsäuretrüsododecylester, Cyclohexan-1,3,5-tricarbonsäuretri-n-undecylester, Cyclohexan-1,3,5-tricarbonsäuretriisododecylester, Cyclohexan-1,3,5-tricarbonsäuretri-n-octadecylester, Cyclohexan-1,3,5-tricarbonsäuretriisooctadecylester, Cyclohexan-1,3,5-tricarbonsäuretrin-eicosylester, Cyclohexan-1,3,5-tricarbonsäuretricyclohexylester.

Cyclohexan-1,2,3-tricarbonsäurealkylester, wie z.B. Cyclohexan-1,2,3-tricarbonsäuremonomethylester, Cyclohexan-1,2,3-tricarbonsäuredimethylester, Cyclohexan-1,2,3-tricarbonsäurediethylester, Cyclohexan-1,2,3-tricarbonsäuredi-n-propylester, Cyclohexan-1,2,3-tricarbonsäuredi-n-butylester, Cyclohexan-1,2,3-tricarhonsäureditert-butylester, Cyclohexan-1,2,3-tricarbonsäurediisobutylester, Cyclohexan-1,2,3-tricarbonsäuremonoglykolester, Cyclohexan-1,2,3-tricarbonsäurediglykolester, Cyclohexan-1,2,3-tricarbonsäuredi-n-octylester, Cyclohexan-1,2,3-tricarbonsäurediisooctylester, Cyclohexan-1,2,3-tricarbonsäuredi-2-ethylhexylester, Cyclohexan-1,2,3-tricarbonsäuredi-n-nonylester, Cyclohexan-1,2,3-tricarbonsäurediisononylester, Cyclohexan-1,2,3-tricarbonsäuredi-n-decylester, Cyclohexan-1,2,3-tricarbonsäurediisodecylester, Cyclohexan-1,2,3-tricarbonsäuredi-n-undecylester, Cyclohexan-1,2,3-tricarbonsäurediisododecylester, Cyclohexan-1,2,3-tricarbonsäuredi-n-octadecylester, Cyclohexan-1,2,3-tricarbonsäurediisooctadecylester, Cyclohexan-1,2,3-tricarbonsäuredi-n-eicosylester, Cyclohexan-1,2,3-tricarbonsäuremonocyclohexylester, Cyclohexan-1,2,3-tricarbonsäuredicyclohexylester, sowie Cyclohexan-1,2,3-tricarbonsäuretrimethylester, Cyclohexan-1,2,3-tricarbonsäuretriethylester, Cyclohexan-1,2,3-tricarbonsäuretri-n-propylester, Cyclohexan-1,2,3-tricarbonsäuretri-n-butylester, Cyclohexan-1,2,3-tricarbonsäuretri-tert-butylester, Cyclohexan-1,2,3-tricarbonsäuretriisobutylester, Cyclohexan-1,2,3-tricarbonsäuretriglykolester, Cyclohexan-1,2,3-tricarbonsäuretri-n-octylester, Cyclohexan-1,2,3-tricarbonsäuretriisooctylester, Cyclohexan-1,2,3-tricarbonsäuretri-2-ethylhexylester, Cyclohexan-1,2,3-tricarbonsäuretri-n-nonylester, Cyclohexan-1,2,3-tricarbonsäuretriisododecylester, Cyclohexan-1,2,3-tricarbonsäuretri-n-undecylester, Cyclohexan-1,2,3-tricarbonsäuretriisododecylester, Cyclohexan-1,2,3-tricarbonsäuretri-n-octadecylester, Cyclohexan-1,2,3-tricarbonsäuretriisooctadecylester, Cyclohexan-1,2,3-tricarbonsäuretrin-eicosylester, Cyclohexan-1,2,3-tricarbonsäuretricyclohexylester.

Cyclohexan-1,2,4,5-tetracarbonsäurealkylester, wie z.B. Cyclohexan-1,2,4,5-tetracarbonsäuremonomethylester, Cyclohexan-1,2,4,5-tetracarbonsäuredimethylester, Cyclohexan-1,2,4,5-tetracarbonsäurediethylester, Cyclohexan-1,2,4,5-tetracarbonsäuredi-n-propylester, Cyclohexan-1,2,4,5-tetracarbonsäuredi-n-butylester, Cyclohexan-1,2,4,5-tetracarbonsäuredi-tert.-butylester, Cyclohexan-1,2,4,5-tetracarbonsäurediisobutylester, Cyclohexan-1,2,4,5-tetracarbonsäuremonoglykolester, Cyclohexan-1,2,4,5-tetracarbonsäurediglykolester, Cyclohexan-1,2,4,5-tetracarbonsäuredin-octylester, Cyclohexan-1,2,4,5-tetracarbonsäurediisooctylester, Cyclohexan-1,2,4,5-tetracarbonsäuredi-2-ethylhexylester, Cyclohexan-1,2,4,5-tetracarbonsäuredin-nonylester, Cyclohexan-1,2,4,5-tetracarbonsäurediisononylester, Cyclohexan-1,2,4,5-tetracarbonsäuredi-n-decylester, Cyclohexan-1,2,4,5-tetracarbonsäurediisodecylester, Cyclohexan-1,2,4,5-tetracarbonsäuredi-n-undecylester, Cyclohexan-1,2,4,5-tetracarbonsäurediisododecylester, Cyclohexan-1,2,4,5-tetracarbonsäuredi-n-octadecylester, Cyclohexan-1,2,4,5-tetracarbonsäurediisooctadecylester, Cyclohexan-1,2,4,5-tetracarbonsäuredi-n-eicosylester, Cyclohexan-1,2,4,5-tetracarbonsäuremonocyclohexylester, Cyclohexan-1,2,4,5-tetracarbonsäuretrimethylester, Cyclohexan-1,2,4,5-tetracarbonsäuretriethylester, Cyclohexan-1,2,4,5-tetracarbonsäuretri-n-propylester, Cyclohexan-1,2,4,5-tetracarbonsäuretri-n-butylester, Cyclohexan-1,2,4,5-tetracarbonsäuretri-tert-butylester, Cyclohexan-1,2,4,5-tetracarbonsäuretrüsobutylester, Cyclohexan-1,2,4,5-tetracarbonsäuretriglykolester, Cyclohexan-1,2,4,5-tetracarbonsäuretri-n-octylester, Cyclohexan-1,2,4,5-tetracarbonsäuretriisooctylester, Cyclohexan-1,2,4,5-tetracarbonsäuretri-2-ethylhexylester, Cyclohexan-1,2,4,5-tetracarbonsäuretri-n-nonylester, Cyclohexan-1,2,4,5-tetracarbonsäuretrüsododecylester, Cyclohexan-1,2,4,5-tetracarbonsäuretri-n-undecylester, Cyclohexan-1,2,4,5-tetracarbonsäuretriisododecylester, Cyclohexan-1,2,4,5-tetracarbonsäuretri-n-octadecylester, Cyclohexan-1,2,4,5-tetracarbonsäuretrüsooctadecylester, Cyclohexan-1,2,4,5-tetracarbonsäuretri-n-eicosylester, Cyclohexan-1,2,4,5-tetracarbonsäuretricyclohexylester, sowie Cyclohexan-1,2,4,5-tetracarbonsäuretetramethylester, Cyclohexan-1,2,4,5-tetracarbonsäuretetraethylester, Cyclohexan-1,2,4,5-tetracarbonsäuretetra-n-propylester, Cyclohexan-1,2,4,5-tetracarbonsäuretetra-n-butylester, Cyclohexan-1,2,4,5-tetracarbonsäuretetra-tert-butylester, Cyclohexan-1,2,4,5-tetracarbonsäuretetraisobutylester, Cyclohexan-1,2,4,5-tetracarbonsäuretetraglykolester, Cyclohexan-1,2,4,5-tetracarbonsäuretetra-n-octylester, Cyclohexan-1,2,4,5-tetracarbonsäuretetraisooctylester, Cyclohexan-1,2,4,5-tetracarbonsäuretetra-2-ethylhexylester, Cyclohexan-1,2,4,5-tetracarbonsäuretetra-n-nonylester, Cyclohexan-1,2,4,5-tetracarbonsäuretetraisododecylester, Cyclohexan-1,2,4,5-tetracarbonsäuretetra-n-undecylester, Cyclohexan-1,2,4,5-tetracarbonsäuretetraisododecylester, Cyclohexan-1,2,4,5-tetracarbonsäuretetra-n-octadecylester, Cyclohexan-1,2,4,5-tetracarbonsäuretetraisooctadecylester, Cyclohexan-1,2,4,5-tetracarbonsäuretetra-n-eicosylester, Cyclohexan-1,2,4,5-tetracarbonsäuretetracyclohexylester.

Anhydride der Cyclohexan-1,2-dicarbonsäure, Cyclohexan-1,2,4-tricarbonsäure, Cyclohexan-1,2,3-tricarbonsäure und Cyclohexan-1,2,4,5-tetracarbonsäure.

Im Sinne der vorliegenden Erfindung als toxikologisch günstig sind darüber hinaus auch die in der PCT/EP 98/08346 offenbarten, im folgenden nochmals aufgelisteten Cyclohexan-1,2-dicarbonsäureester zu bewerten:
Cyclohexan-1,2-dicarbonsäuredi(isopentyl)ester, erhältlich durch Hydrierung von Di(isopentyl)phthalat mit der Chemical Abstracts Registry Nummer (im folgenden: CAS Nr.) 84777-06-0;
Cyclohexan-1,2-dicarbonsäuredi(isoheptyl)ester, erhältlich durch Hydrierung von Di(isoheptyl)phthalat mit der CAS Nr. 71888-89-6;
Cyclohexan-1,2-dicarbonsäuredi(isononyl)ester, erhältlich durch Hydrierung eines Di(isononyl)phthalats mit der CAS Nr. 68515-48-0;
Cyclohexan-1,2-dicarbonsäuredi(isononyl)ester, erhältlich durch Hydrierung eines Di(isononyl)phthalats mit der CAS Nr. 28553-12-0, basierend auf n-Buten;
Cyclohexan-1,2-dicarbonsäuredi(isononyl)ester, erhältlich durch Hydrierung eines Di(isononyl)phthalats mit der CAS Nr. 28553-12-0 basierend auf Isobuten;
ein 1,2-Di-C₉-Ester der Cyclohexandicarbonsäure, erhältlich durch Hydrierung eines Di(nonyl)phthalats mit der CAS Nr. 68515-46-8;
ein Cyclohexan-1,2-dicarbonsäuredi(isodecyl)ester erhältlich durch Hydrierung eines Di(isodecyl)phthalats mit der CAS Nr. 68515-49-1;
ein 1,2-Di-C₇₋₁₁-Ester der Cyclohexandicarbonsäure, erhältlich durch Hydrierung des entsprechenden Phthalsäureesters mit der CAS Nr. 68515-42-4;
ein 1,2-Di-C₇₋₁₁-Ester der Cyclohexandicarbonsäure, erhältlich durch Hydrierung der Di-C₇₋₁₁-Phthalate mit folgenden CAS Nr.
111 381-89-6,
111 381 90-9,
111 381 91-0,
68515-44-6,
68515-45-7 und
3648-20-7;
ein 1,2-Di-C₉₋₁₁-Ester der Cyclohexandicarbonsäure, erhältlich durch Hydrierung eines Di-C₉₋₁₁-Phthalats mit der CAS Nr. 98515-43-5;
ein 1,2-Di(isodecyl)cyclohexandicarbonsäureester, erhältlich durch Hydrierung eines Di(isodecyl)phthalats, das hauptsächlich aus Di-(2-propylheptyl)phthalt besteht;
ein 1,2-Di-C₇₋₉-Cyclohexandicarbonsäureester, erhältlich durch Hydrierung des entsprechenden Phthalsäureesters der verzweigtkettige oder lineare C₇₋₉-Alkylestergruppen aufweist; entsprechende beispielsweise als Ausgangsprodukte verwendbare Phthalate haben die folgende CAS Nr.:
   Di-C_{7,9}-Alkylphthalat mit der CAS Nr. 111 381-89-6;
   Di-C₇-Alkylphthalat mit der CAS Nr. 68515-44-6; und
   Di-C₉-Alkylphthalat mit der CAS Nr. 68515-45-7.

Der Inhalt der PCT/EP 98/08346, der sich u.a. auf diese soeben aufgelisteten Verbindungen und die Herstellung von Benzolpolycarbonsäuren unter Verwendung spezieller Makroporen aufweisender Katalysatoren bezieht, wird durch Bezugnahme vollständig in die vorliegende Anmeldung einbezogen.

Des weiteren sind auch die Hydrierungsprodukte der kommerziell erhältlichen Benzolcarbonsäureester mit den Handelsnamen Jayflex DINP (CAS Nr. 68515-48-0), Jayflex DIDP (CAS Nr. 68515-49-1), Palatinol 9-P, Vestinol 9 (CAS Nr. 28553-12-0), TOTM-1 (CAS Nr. 3319-31-1), Linplast 68-TM und Palatinol N (CAS Nr. 28553-12-0) als toxikologisch günstig im Sinne der vorliegenden Erfindung zu bewerten.

Die im Sinne der vorliegenden Erfindung toxikologisch als günstig zu bewertenden Cyclohexanpolycarbonsäuren und Derivate davon lassen sich in alle dem Fachmann bekannten Kunststoffen, insbesondere in Massenkunststoffen wie beispielsweise PVC, PVB, sowie PVAc einsetzen.

Im folgenden soll nunmehr die Erfindung anhand eines Ausführungsbeispiels näher erläutert werden.

### BEISPIEL

Nach oraler Verabreichung von 1000 mg / kg Körpergewicht der Prüfsubstanz Cyclohexan-1,2-dicarbonsäurediisononylester über 14 Tage per Schlundsonde wurden bei weiblichen Wistar-Ratten die in Tabelle 1 aufgelisteten Ergebnisse gefunden.

**Tabelle 1: Ergebnisse der Bestimmung des absoluten und relativen Anstiegs des Lebergewichts sowie der Bestimmung der Palmitoyl-CoA Oxidase-Aktivität in weiblichen Wistar-Ratten in (a) der Kontrollgruppe (10 unbehandelte Tiere) und (b) der Versuchsgruppe (10 Tiere, die über eine Dauer von mindestens 14 Tagen täglich eine orale Dosis von 1000 mg/kg Körpergewicht der Prüfsubstanz Cyclohexan-1,2-dicarbonsäurediisononylester per Schlundsonde erhalten haben).**

| Parameter | Gruppe 0 | Gruppe 1 (n = 10) |
|---|---|---|
| | (Kontrollgruppe; n = 10) | (Versuchsgruppe: täglich 1000 mg Prüfsubstanz / kg Körpergewicht per Schlundsonde) |
| | [Mittelwert ± Standardabweichung] | [Mittelwert ± Standardabweichung] |
| Körpergewicht [g] | 400,3 ± 29,7 | 398,9 ± 28,5 |
| Absolutes Lebergewicht [g] | 19,21 ± 2,64 | 19,64 ± 1,77 |
| Relatives Lebergewicht, bezogen auf Körpergewicht [%] | 4,79 ±0,37 | 4,93 ± 0,41 |
| Palmitoyl-CoA Oxidase [mU/mg Protein] | 6,20 ± 0,38 | 7,24 ± 0,87* |

| | | |
|---|---|---|
| * Statistisch signifikante Veränderung nach Mann-Whitney U-Test (vgl. beispielsweise Hollander, M. und Wolfe, D.A. (1973), Nonparametric Statistical Methods, John Wiley and Sons Inc., N.Y), toxikologisch jedoch nicht relevant. | | |

Im Zusammenhang mit der Bestimmung des absoluten bzw. relativen Lebergewichts erfolgte die statistische Auswertung nach dem Dunnett-Test, die Bestimmung der spezifischen Aktivität der cyanid-insensitiven Palmitoyl-CoA Oxidase erfolgte nach Lazarow (1981), Enzymology 72, 315-319.

## Patentansprüche

1. Verwendung einer Cyclohexanpolycarbonsäure oder eines Derivats davon oder eines Gemischs aus zwei oder mehr davon, welche(s) im Tierversuch mit Nagern bei einer täglichen oralen Dosierung von 1000 mg / kg Körpergewicht der entsprechenden Cyclohexanpolycarbonsäure oder des Derivats davon oder des Gemischs aus zwei oder mehr davon per Schlundsonde über die Dauer von mindestens 14 Tagen im Vergleich zu unbehandelten Kontrolltieren weder zu einem signifikanten Anstieg des Lebergewichtes nach der Behandlung noch zu einer Verdoppelung der im Leberhomogenat gemessenen spezifischen Aktivität der cyanid-insensitiven Palmitoyl-CoA Oxidase führt, als Weichmacher zur Herstellung toxikologisch günstig zu bewertender Kunststoffe.

2. Verwendung nach Anspruch 1, **dadurch gekennzeichnet, daß** die Cyclohexanpolycarbonsäure oder das Derivat davon oder das Gemisch aus zwei oder mehr davon ausgewählt wird aus der Gruppe bestehend aus kernhydrierten Mono- und Dialkylestern der Phthalsäure, Isophthalsäure und Terephthalsäure, kernhydrierten Mono-, Di- und Trialkylestern der Trimellitsäure, der Trimesinsäure und der Hemimillitsäure, Mono-, Di-, Tri- und Tetraalkylestern der Pyrromeliitsäure, wobei die Alkylgruppen linear oder verzweigt sein könne und jeweils 1 bis 30 Kohlenstoffatome aufweisen, kernhydrierten Anhydriden der Phthalsäure, Trimellitsäure, Trimesinsäure und Hemimellitsäure, Pyrromellitsäuredianhydrid und Gemischen aus zwei oder mehr davon.

3. Verwendung nach Anspruch 1, **dadurch gekennzeichnet, daß** die Cyclohexanpolycarbonsäure oder das Derivat davon oder das Gemisch aus zwei oder mehr davon ausgewählt wird aus der Gruppe bestehend aus:
Cyclohexan-1,2-dicarbonsäuredi(isopentyl)ester, erhältlich durch Hydrierung von Di(isopentyl)phthalat mit der Chemical Abstracts Registry- Nummer (im folgenden: CAS Nr.) 84777-06-0;
Cyclohexan-1,2-dicarbonsäuredi(isoheptyl)ester, erhältlich durch Hydrierung von Di(isoheptyl)phthalat mit der CAS Nr. 71888-89-6;
Cyclohexan-1,2-dicarbonsäuredi(isononyl)ester, erhältlich durch Hydrierung eines Di(isononyl)phthalts mit der CAS Nr. 68515-48-0;
Cyclohexan-1,2-dicarbonsäuredi(isononyl)ester, erhältlich durch Hydrierung eines Di(isononyl)phthalats mit der CAS Nr. 28553-12-0, basierend auf n-Buten;
Cyclohexan-1,2-dicarbonsäuredi(isononyl)ester, erhältlich durch Hydrierung eines Di(isononyl)phthalats mit der CAS Nr. 28553-12-0, basierend auf Isobuten;
ein 1,2-Di-C₉-Ester der Cyclohexandicarbonsäure, erhältlich durch Hydrierung eines Di(nonyl)phthalts mit der CAS Nr. 68515-46-8;
ein Cyclohexan-1,2-dicarbonsäuredi(isodecyl)ester erhältlich durch Hydrierung eines Di(isodecyl)phthalais mit der CAS Nr. 68515-49-1;
ein 1,2-Di-C₇₋₁₁-Ester der Cyclohexandicarbonsäure, erhältlich durch Hydrierung des entsprechenden Phthalsäureesters mit der CAS Nr. 68515-42-4;
ein 1,2-Di-C₇₋₁₁-Ester der Cyclohexandicarbonsäure, erhältlich durch Hydrierung der Di-C₇₋₁₁-Phthalate mit folgenden CAS Nr.:
111 381-89-6,
111 381 90-9,
111 381 91-0,
68515-44-6,
68515-45-7 und
3648-20-7
ein 1,2-Di-C₉₋₁₁-Ester der Cyclohexandicarbonsäure, erhältlich durch Hydrierung eines Di-C₉₋₁₁-Phthalats mit der CAS Nr. 98515-43-5;
ein 1,2-Di(isodecyl)cyclohexandicarbonsäureester, erhältlich durch Hydrierung eines Di(isodecyl)phthalats, das hauptsächlich aus Di-(2-propylheptyl)phthalat besteht;
ein 1,2-Di-C₇₋₉-Cyclohexandicarbonsäureester, erhältlich durch Hydrierung des entsprechenden Phthalsäureesters, der verzweigtkettige oder lineare C₇₋₉-Alkylestergruppen aufweist.

## Claims

1. The use of a cyclohexanepolycarboxylic acid or of a derivative thereof or of a mixture made from two or more thereof, which when tested on rodents at a daily oral dose of 1000 mg/kg of body weight via a stomach tube of the appropriate cyclohexanepolycarboxylic acid or of the derivative thereof or of the mixture made from two or more thereof over a period of at least 14 days causes no significant rise in the liver weight after the treatment and does not cause a doubling of the specific activity of the cyanide-insensitive palmitoyl-CoA oxidase, measured in the liver homogenate, compared with untreated control animals, as a plasticizer for preparing toxicologically advantageous plastics.

2. The use according to claim 1, wherein the cyclohexanepolycarboxylic acid or the derivative thereof or the mixture made from two or more thereof is selected from the group consisting of ring-hydrogenated mono- and dialkyl esters of phthalic acid, isophthalic acid or terephthalic acid, ring-hydrogenated mono-, di- and trialkyl esters of trimellitic acid, of trimesic acid or of hemimellitic acid, mono-, di-, tri- and tetraalkyl esters of pyromellitic acid, where the alkyl groups may be linear or branched and in each case have from 1 to 30 carbon atoms, ring-hydrogenated anhydrides of phthalic acid, trimellitic acid, trimesic acid or hemimellitic acid, pyromellitic dianhydride and mixtures made from two or more of these.

3. The use according to claim 1, wherein the cyclohexanepolycarboxylic acid or the derivative thereof or the mixture made from two or more thereof is selected from the group consisting of:
cyclohexane-1,2-dicarboxylic acid di(isopentyl) ester obtainable by hydrogenating di(isopentyl) phthalate with Chemical Abstracts Registry Number (hereinafter: CAS No.) 84777-06-0;
cyclohexane-1,2-dicarboxylic acid di(isoheptyl) ester obtainable by hydrogenating di(isoheptyl) phthalate with CAS No. 71888-89-6;
cyclohexane-1,2-dicarboxylic acid di(isononyl) ester obtainable by hydrogenating a di(isononyl) phthalate with CAS No. 68515-48-0;
cyclohexane-1,2-dicarboxylic acid di(isononyl) ester obtainable by hydrogenating a di(isononyl) phthalate with CAS No. 28553-12-0, based on n-butene;
cyclohexane-1,2-dicarboxylic acid di(isononyl) ester obtainable by hydrogenating a di(isononyl) phthalate with CAS No. 28553-12-0, based on isobutene;
a 1,2-di-C₉ ester of cyclohexanedicarboxylic acid obtainable by hydrogenating a di(nonyl) phthalate with CAS No. 68515-46-8;
a cyclohexane-1,2-dicarboxylic acid di(isodecyl) ester obtainable by hydrogenating a di(isodecyl) phthalate with CAS No. 68515-49-1;
a 1,2-di-C₇₋₁₁ ester of cyclohexanedicarboxylic acid obtainable by hydrogenating the corresponding phthalic ester with CAS No. 68515-42-4;
a 1,2-di-C₇₋₁₁ ester of cyclohexanedicarboxylic acid obtainable by hydrogenating the di-C₇₋₁₁ phthalates with the following CAS Nos:
111 381-89-6,
111 381 90-9,
111 381 91-0,
68515-44-6,
68515-45-7 and
3648-20-7;
a 1,2-di-C₉₋₁₁ ester of cyclohexanedicarboxylic acid obtainable by hydrogenating a di-C₉₋₁₁ phthalate with CAS No. 98515-43-5;
a 1,2-di(isodecyl) ester of cyclohexanedicarboxylic acid, obtainable by hydrogenating a di(isodecyl) phthalate composed mainly of di(2-propylheptyl) phthalate;
a 1,2-di-C₇₋₉ ester of cyclohexanedicarboxylic acid, obtainable by hydrogenating the corresponding phthalic ester which has branched-chain or linear C₇₋₉-alkyl ester groups.

## Revendications

1. Utilisation d'un acide cyclohexanepolycarboxylique ou d'un dérivé de celui-ci ou d'un mélange de deux ou plusieurs d'entre eux qui, dans un essai sur animal avec des rongeurs, lors d'un dosage oral journalier de 1000 mg/kg de poids de corps de l'acide cyclohexanepolycarboxylique correspondant ou de son dérivé ou du mélange de deux ou plusieurs d'entre eux par une sonde oesophagienne sur une durée d'au moins 14 jours, ne donne lieu, en comparaison des animaux témoins non traités, ni à une augmentation significative du poids du foie après le traitement, ni à un doublement de l'activité spécifique mesurée dans un homogénat du foie de la palmitoyl-CoA oxydase insensible au cyanure, comme plastifiant pour la préparation de substances synthétiques dont la valorisation est favorable sur le plan toxicologique.

2. Utilisation suivant la revendication 1, **caractérisée en ce que** l'acide cyclohexanepolycarboxylique ou son dérivé ou le mélange de deux ou de plusieurs d'entre eux est choisi parmi le groupe constitué des esters monoalkyliques et dialkyliques hydrogénés sur le noyau de l'acide phtalique, de l'acide isophtalique et de l'acide téréphtalique, des esters monoalkyliques, dialkyliques et trialkyliques hydrogénés sur le noyau de l'acide trimellitique, de l'acide trimésique et de l'acide hémimellitique, des esters monoalkyliques, dialkyliques, trialkyliques et tétraalkyliques de l'acide pyrromellitique, où les groupes alkyle peuvent être linéaires ou ramifiés et présentent chacun 1 à 30 atomes de carbone, des anhydrides hydrogénés sur le noyau de l'acide phtalique, de l'acide trimellitique, de l'acide trimésique et de l'acide hémimellitique, du dianhydride d'acide pyrromellitique et de mélanges de deux ou plusieurs d'entre eux.

3. Utilisation suivant la revendication 1, **caractérisée en ce que** l'acide cyclohexanepolycarboxylique ou son dérivé ou le mélange de deux ou plusieurs d'entre eux est choisi parmi le groupe constitué du :
cyclohexane-1,2-dicarboxylate de di(isopentyle), que l'on peut obtenir par hydrogénation de phtalate de di(isopentyle) ayant le numéro de registre du Chemical Abstracts (dans la suite : CAS n°) 84777-06-0,
cyclohexane-1,2-dicarboxylate de di(isoheptyle), que l'on peut obtenir par hydrogénation de phtalate de di(isoheptyle) ayant le CAS n° 71888-89-6,
cyclohexane-1,2-dicarboxylate de di(isononyle), que l'on peut obtenir par hydrogénation d'un phtalate de di(isononyle) ayant le CAS n° 68515-48-0,
cyclohexane-1,2-dicarboxylate de di(isononyle), que l'on peut obtenir par hydrogénation d'un phtalate de di(isononyle) ayant le CAS n° 28553-12-0, à base de n-butène,
cyclohexane-1,2-dicarboxylate de di(isononyle), que l'on peut obtenir par hydrogénation d'un phtalate de di(isononyle) ayant le CAS n° 28553-12-0, à base d'isobutène,
un 1,2-di-C₉-ester de l'acide cyclohexanedicarboxylique, que l'on peut obtenir par hydrogénation d'un phtalate de di(nonyle) ayant le CAS n° 68515-46-8,
un cyclohexane-1,2-dicarboxylate de di(isodécyle), que l'on peut obtenir par hydrogénation d'un phtalate de di(isodécyle) ayant le CAS n° 68515-49-1,
un 1, 2-di-C₇₋₁₁-ester de l'acide cyclohexanedicarboxylique, que l'on peut obtenir par hydrogénation de l'ester d'acide phtalique correspondant ayant le CAS n° 68515-42-4,
un 1,2-di-C₇₋₁₁-ester de l'acide cyclohexanedicarboxylique, que l'on peut obtenir par hydrogénation du di-C₇₋₁₁-phtalate ayant les CAS n° suivants :
111 381-89-6,
111 381 90-9,
111 381 91-0,
68515-44-6,
68515-45-7 et
3648-20-7,
un 1,2-di-C₉₋₁₁-ester de l'acide cyclohexanedicarboxylique, que l'on peut obtenir par hydrogénation d'un di-C₉₋₁₁-phtalate ayant le CAS n° 98515-43-5,
un ester d'acide 1,2-di(isodécyl)cyclohexanedicarboxylique, que l'on peut obtenir par hydrogénation d'un phtalate de di(isodécyle), qui est constitué principalement de phtalate de di(2-propylheptyle),
un ester d'acide 1,2-di-C₇₋₉-cyclohexanedicarboxylique, que l'on peut obtenir par hydrogénation de l'ester d'acide phtalique correspondant, qui présente des groupes esters alkyliques en C₇₋₉ linéaires ou ramifiés.
